# EUROPEAN PATENT SPECIFICATION

(11) **EP 1 807 513 B1**
(45) Date of publication and mention of the grant of the patent: **17.08.2016**
(21) Application number: 05797837.1
(22) Date of filing: 27.10.2005
(51) Int. Cl.: C12N 9/96, C12N 9/02, C12N 9/20, C12P 19/04

(54) **METHOD FOR THE PREPARATION OF CROSS-LINKED ENZYME AGGREGATES WITH IMPROVED PROPERTIES**
VERFAHREN ZUR HERSTELLUNG QUERVERNETZTER ENZYMAGGREGATE MIT VERBESSERTEN EIGENSCHAFTEN
PROCÉDÉ DE PRÉPARATION D'AGRÉGATS D'ENZYMES RÉTICULÉES PRÉSENTANT DES PROPRIÉTÉS AMÉLIORÉES

(30) Priority: 28.10.2004 NL 1027360
(43) Date of publication of application: 18.07.2007
(73) Proprietor: Clea Technologies BV, 2628 BL, Delft (NL)
(72) Inventor: SCHOEVAART, Willem, Robert, Klaas, 2623 PG Delft (NL); VAN LANGEN, Lukas, Michael, 2623 HA Delft (NL); VAN DEN DOOL, Ronald, Tako, Marinus, 4102 KR Culemborg (NL); BOUMANS, Johannes, Wilhelmus, Leonardus, 1191 RH Ouderkerk Aan De Amstel (NL)
(74) Representative: van der Kloet-Dorleijn, G.W.F.
(86) International application number: PCT/NL2005/000767
(87) International publication number: WO 2006/046865

(56) References cited:
- EP-A- 1 088 887
- EP-A- 1 418 231
- WO-A-99/12959
- US-A- 5 437 993
- VUOLANTO A: "Cross-linked protein crystal technology in bioseparation and biocatalytic applications" 20 August 2004 (2004-08-20), HELSINKI UNIVERSITY OF TECHNOLOGY , ESPOO, FINLAND , XP002330803 page 10 - page 13, paragraph 1
- LOPEZ-SERRANO P ET AL: "CROSS-LINKED ENZYME AGGREGATES WITH ENHANCED ACTIVITY: APPLICATION TO LIPASES" BIOTECHNOLOGY LETTERS, KEW, SURREY, GB, vol. 24, no. 16, August 2002 (2002-08), pages 1379-1383, XP009003775 ISSN: 0141-5492
- SCHOEVAART R ET AL: "Preparation, optimization, and structures of cross-linked enzyme aggregates (CLEAs)" BIOTECHNOLOGY AND BIOENGINEERING, vol. 87, no. 6, 20 September 2004 (2004-09-20), pages 754-762, XP002394547 ISSN: 0006-3592
- POZNANSKY M: "Soluble enzyme-albumin conjugates: New possibilities for enzyme replacement therapy" METHODS IN ENZYMOLOGY, vol. 137, no. d, 1988, pages 566-574, XP002330802

## Description

The present invention relates to a method for the preparation of cross-linked enzyme aggregates (CLEAs).

Methods for the preparation of cross-linked enzyme aggregates are known in the prior art. Such a method is, for example, described in EP 1088887 A1, and in Schoevaart et al, Biotechnology and Bioengineering, vol 87, no. 6, September 20, 2004, which disclose the preparation of CLEAs comprising the precipitation of the enzyme, followed by the addition of aldehyde groups and cross-linking the aggregate.

The possibility of varying properties afforded by such known methods is limited, and consequently such methods often result in cross-linked enzyme aggregates with properties, in particular activity and colloidal behaviour, that are not optimal for the ultimate purpose of the cross-linked enzyme aggregates.

Further, WO 99/12959 discloses the preparation of cross-linked enzyme crystals (so-called CLECs), comprising the cross-linking with an amine of enzyme crystals on which aldehyde groups have been introduced by oxidation.

A similar process of oxidation of the enzyme crystals followed by cross-linking is known from A. Vuolanto, Techn. Biochem.Report 1/2004, Espoo 2004, Helsinki University of Technology.

Lopez-Serrano et al, Biotechnology Letters 24: 1379-1383, 2002, indicate the advantage of using enzyme aggregates instead of enzyme crystals as starting point for cross-linked enzyme compositions.

It is therefore an object of the present invention to provide a method for the preparation of cross-linked enzyme aggregates that allows for use of a wider range of reagents and thus creates the possibility to obtain enzyme aggregates with improved properties, in particular with regard to activity and colloidal behaviour.

To this end the method according to the present invention is characterized by the following steps:
i. generating aldehyde groups on enzyme molecules that may be dissolved in a solvent, by the addition of an oxidant selected from the group of periodates of alkaline-earth metals;
ii. precipitating the enzyme molecules using a suitable precipitation agent;
iii. cross-linking the precipitated enzyme molecules provided with aldehyde groups, using an amine composition, yielding cross-linked enzyme aggregates with improved properties.

Surprisingly, it was shown that by using the method according to the present invention, enzyme aggregates could be obtained with improved properties, in particular improved activity and colloidal behaviour.

This is made possible by the fact that the cross-linking agent consists of two components, namely an aldehyde component on the enzyme and an gamine component to be added separately, which together can be applied in many different combinations.

Step i. of the method according to the present invention is preferably carried out by the addition of an aldehyde compound. This may be a mono-, di- or polyaldehyde, wherein a monoaldehyde should have at least one reactive group capable of reacting with an enzyme, forming one or several aldehyde groups on the enzyme.

Such aldehydes are shown to be cheap and effective agents for the generation of aldehyde groups on an enzyme molecule.

Step i. in the method according to the invention is carried out by the addition of an oxidant, selected from the group of periodated of alkaline-earth metals, wherein sodiumperiodate (NaIO₄) is most preferred.

Carrying out step i. of the method according to the invention (the generation of aldehyde groups on the enzyme molecules.), by adding a suitable oxidant such as NaIO₄, has the advantage that aldehyde groups are generated on specific sites on the enzyme and that generally the enzymatic activity is preserved. Moreover, any salts that may be formed can simply be washed away during an optional wash step.

It is also preferred to add a reducing agent in step iii.

The addition of a reducing agent causes free aldehyde groups of the enzyme to bond with the added amines.

The addition of a reducing agent in step iii. of the method according to the invention has the advantage that cross-linking occurs in a few minutes so that, compared with other methods, the preparation time is shorter. In addition, aldehydes that have not reacted with an amine are inactivated, preventing them from reacting at a later stage. This latter aspect is important because it prevents the particle size of the cross-linked enzyme aggregates from becoming larger than 10 to 50 micrometres. Particles becoming too large have an adverse effect on the activity. Besides a reduction agent particle increase can be avoided by the addition of alkoxysilanes. Furthermore, by choosing a more hydrophobic or a more hydrophilic silicate the hydrophobicity of the CLEA can be controlled.

The reducing agent is preferably selected from the group of NaCNBH₃ or NaBH₄.

These reducing agents were shown to be especially suitable.

The alkoxysilanes are preferably selected from the group of (MeO)₄Si, (EtO)₄Si, Me(MeO)₃Si and Propyl(MeO)₃Si.

In step iii. of the method according to the present invention it is preferred to use ammonia as amine compound.

Until now it was common practice only to consider di- and polyamine compounds as cross-linking agents for cross-linked enzyme aggregates. However, the inventors have now found that using ammonia as cross-linking agent provides cross-linked enzyme aggregates with favourable properties. When using ammonia as amine compound, the cross-linked enzyme aggregates that are obtained are generally not coloured. The particles are uniform, and the small particles that are formed can be instantly resuspended in water without extra mechanical treatment. This yields good activity.

It is further preferred to use a diamine as amine compound in step iii. of the method.

Until now it was common practice to associate the length of the diamine as cross-linking agent with the activity and the cross-linked enzyme particles obtained thereby.

Surprisingly, it has now been found that with the method of the invention aldehyde groups are generated, without the occurrence of cross-linking, so that in order to obtain particular physical properties of the cross-linked enzyme aggregates the di- or polyamide can be specifically selected.

For example, the introduction of acidic groups (e.g. using the amino acid lysine) gives the cross-linked enzyme aggregate a negative charge in a basic environment, and the introduction of basic groups (e.g. using polyethylenediamines such as pentaethylenehexamine) gives the cross-linked enzyme aggregate a positive charge in an acidic environment. These treatments result in different colloidal behaviour. By applying the modification that is the most favourable for the enzyme, the activity can be maintained or increased. It is also possible for apolar groups (e.g. using xylenediamine, polyxylylenediamine, 1,3-propanediamine, hexanediamine) or polar groups (e.g. 1,3-diamino-2-propanol) to be incorporated, depending on the solvent that is used in the intended application. The amine may also be obtained from the protein itself, in the form of amine-comprising side groups of amino acids, such as lysine.

It is further preferred for the amine compound in step ii. of the method according to the present invention to be derived from the precipitation agent of step i.

This has the advantage that in order to perform step iii. it is not necessary to separately add an amine compound so that fewer operations are necessary and, due to the higher amine concentration, fewer reactive amines are needed with all the consequential time and cost savings.

The precipitation agent is preferably an ammonium compound and the amine compound ammonia, the method being performed at a pH of 8-9.5. When using di- or polyamines it is preferred not to use ammonium salt but polyethylene glycol or another amine-free solvent.

It is still more preferred for the ammonium compound to be ammonium sulphate. When using ammonium sulphate and glutaraldehyde no spontaneous cross-linking takes place if the pH is kept between 8 and 9.5. At a lower pH, spontaneous cross-linking does occur before the reduction step can take place so that cross-linked aggregates are obtained having very strongly deviating properties such as discolouring and large particles.

When using di- or polyamines the pH may be varied between 1 and 14, preferably between 4 and 10 and most optimally between 6 and 8.

It has further been found that the enzyme molecules in the method according to the present invention can be precipitated with a suitable precipitation agent (step i.) preceding the generation of aldehyde groups on the enzyme molecules (step ii.).

This aspect of the method may be employed advantageously, for example, when NaIO₄ is used to introduce aldehyde groups on the protein. NaIO₄ dissolves very well in aqueous solvents (containing the enzymes before precipitation), whereas it dissolves poorly in the usual precipitation agents. This reduces its activity after aggregation and avoids disruption of further steps in the process.

The particle size of the cross-linked enzyme aggregates obtained using the method according to the present invention may in some cases be too small for filtering with the aid of, for example, conventional glass filters.

It is therefore advantageous if the possibility exists to enlarge.the particle size of the cross-linked enzyme aggregates.

To this end the method according to the present invention is characterised in a preferred embodiment by the addition in step i. of a carrier material.

The carrier material is preferably silica, Sepabeads® or another known enzyme carrier.

According to the prior art, enzymes and cross-linked enzyme aggregates are generally washed with a buffer solution. It is generally accepted that washing with a buffer is best for the quality of the aggregates.

In contrast, it has now been found that it is advantageous if after step iii. of the method according to the present invention, the cross-linked enzyme aggregates are washed with demineralised water, followed by a drying step.

Salts and other substances in solution are thus effectively washed away, resulting in increased activity in organic media and in ionic liquids.

Subsequently, it is preferred for the drying step to be carried out by treatment with an organic solvent, preferably selected from the group of water-miscible ,solvents such as acetone, alcohols and ionic liquids. The water-miscible solvent may optionally be washed away with a more volatile solvent (such as diethyl ether) in order to expedite the drying process.

It has been shown that the activity in organic media and ionic liquids of cross-linked enzyme aggregates prepared by using the method according to the present invention is improved. Acetone, water-miscible ethers, alcohols and ionic liquids have been found to be inexpensive and effective solvents.

The organic solvent may subsequently be removed by the addition of ether, followed by evaporation.

The addition of ether followed by evaporation dries very efficiently. Optionally, a known amount of water may be added to the cross-linked aggregate if this appears to enhance the activity. This may, for example, be carried out by adding acetone to a known percentage of water and subsequently removing the acetone with ether. A small amount of water in the cross-linked aggregate may result in enhanced activity, if the medium in which the cross-linked aggregate is ultimately used is a water-free medium.

In another aspect, the present invention relates to enzyme aggregates that can be obtained in accordance with the methods of the present invention.

Compared with aggregates prepared in accordance with prior art methods, the activity and colloidal behaviour of these aggregates is improved.

Favourable results were obtained when the enzyme molecules were selected from the group of laccase molecules and lipase molecules, but the method according to the present invention may also be applied to other enzymes such as proteases, esterases, oxynitrilase, nitrilase, aminoacylase, penicillin acylase, oxidases, reductases. Especially, lipases and esterases are often used under "dry" conditions, so that drying is very important. Too much water can cause a disruption in the reaction catalysed by the enzyme.

Another favourable feature of cross-linked enzyme aggregates, apart from an enhanced activity, is an improvement of the stability. An important technical limitation of the use of the enzyme laccase for the oxidation of carbohydrates like starch (as known from, for example, WO 0050621) is the fact that during the reaction, the enzyme laccase is also oxidised by the additive 2,2,6,6-tetramethyl-1-piperidinyloxy (TEMPO). As a consequence, the enzyme becomes inactivated after a period of time. This means that during the oxidation process, fresh laccase must be added continuously. For economical reasons, this process can hardly compete with the present-day technology, despite the obvious environmental advantages.

By making a cross-linked aggregate from laccase, the enzyme is better protected against oxidation. The life span during the oxidation of starch is extended, so that the process becomes cost-effective.

Because a free enzyme is unable to penetrate into the cross-linked enzyme aggregate, which is only accessible to smaller molecules, enzymes are in the cross-linked, aggregated condition protected against attack from degrading enzymes such as proteases. This advantage is shown, for example, in the case of laccase enzymes. Although the dissolved enzyme composition shows protease as well as laccase activity, no protease activity can be detected in the cross-linked enzyme aggregate. Compared to the free laccase composition, the stability of the laccase enzyme molecules is much improved in cross-linked enzyme aggregates. An extra purification step of the laccase enzyme molecules is thus rendered superfluous.

For the application of the laccase in the oxidation of starch, this advantage is exhibited in another way. Apart from laccase and protease activity, the dissolved enzyme composition also exhibits amylase activity. Amylase degrades starch into smaller fragments. This is undesirable because smaller starch fragments oxidised by laccase have much poorer properties than starch that is not degraded by amylase and oxidised by laccase. In the cross-linked aggregated form the amylase activity - similar to the protease activity - was shown to be greatly reduced. Thus starch is also too large to penetrate into the cross-linked enzyme aggregate, which normally results in hydrolysis into smaller fragments, leading to undesirable product characteristics. Here also, an extra purification step is superfluous.

The invention will now be elucidated by way of the following, non-limiting examples.

### EXAMPLES

### Example 1: Laccase activity

Laccase (Trametes versicolor, Wacker Chemie) activity was measured by dissolving an amount of laccase enzyme in 40 mM sodium acetate buffer pH 4.5. 100 mg 2,2-azinobis(3-ethylbenzthiazoline-6-sulphonate) (ABTS) was dissolved in 20 ml of the same buffer and 200 µl of this solution was mixed with 800 µl of the laccase solution and incubated at 25°C. The extinction change at 420 nm was used to determine the laccase activity. One unit of activity (U) is defined as the amount of enzyme inducing a change in extinction of 0.027 dE⁴²⁰ per minute in a reaction volume of 1 ml.

### Example 2: Preparation of the nitrosonium salt of 2,2,6,6-tetramethyl-1-piperidinyloxy (TEMPO) using laccase

A solution of TEMPO-nitrosonium ion was prepared as follows using laccase: 6.9 g TEMPO was dissolved in 1 litre demineralised water. 200 mg laccase from Trametes versicolor (Wacker) was suspended in 20 ml demineralised water. After stirring the enzyme solution for 10 minutes, the supernatant (centrifugation 5 minutes at 1500xg) was desalted using a P6 column. The desalted material was added to the TEMPO solution. After approximately 150 minutes under pH stat conditions at pH 5, room temperature, while aerating using an aerator, 91% of the TEMPO was converted into nitrosonium, as was apparent from the consumption of 100.8 ml HCL (0.4 N) and a colour shift from yellow to a more orange tint (the ratio E480/E430 increased from approximately 0.3 to 1.4).

### Example 3: Diamine selection and optimisation

25 mg laccase (Trametes versicolor, Wacker Chemie) was dissolved in 1 ml 100 mM sodium acetate buffer pH 4.5 at 4°C. After centrifugation, 0.9 g polyethylene glycol (8000k). was added to the supernatant at room temperature. After stirring for 30 minutes, chilled (4°C) glutaraldehyde (25% solution) was added to a final concentration of ten times (50-150 mM) that of the diamine. The solution thus obtained was stirred for 5 minutes at room temperature. Subsequently, a selected diamine (as 100 mM solution, pH 4.5) was added all at once, having different final concentrations (5-15 mM). The mixture was stirred slowly for 3 hours at room temperature.

**Table 1. Diamine selection and optimisation**

| **mM diamine** | | | **mM glutaraldehyde** | **% CLEA* activity yield** | **U / mg** | **appearance of the CLEA* suspension** |
|---|---|---|---|---|---|---|
| **1** | 5 | PDA | 50 | 45 | 3,89 | flocculent, precipitates quickly |
| **2** | 10 | PDA | 100 | 39 | 3,38 | |
| **3** | 15 | PDA | 150 | 33 | 2,88 | |
| **4** | 5 | HMDA | 50 | 63 | 4,14 | powdery, precipitates quickly |
| **5** | 10 | HMDA | 100 | 47 | 4,03 | |
| **6** | 15 | HMDA | 150 | 36 | 3,09 | |
| **7** | 5 | PEHA | 50 | 48 | 4,20 | flocculent, precipitates very slowly |
| **8** | 10 | PEHA | 100 | 34 | 3,94 | |
| **9** | 15 | PEHA | 150 | 30 | 2,55 | |
| **10** | 5 | PXDA | 50 | 45 | 3,87 | large particles |
| **11** | 10 | PXDA | 100 | 31 | 2,67 | |
| **12** | 15 | PXDA | 150 | 30 | 2,56 | |

| | | | | | | |
|---|---|---|---|---|---|---|
| *CLEA= cross-linked enzyme aggregate PDA= 1,3-propanediamine; HMDA = 1,6-hexamethylenediamine; PEHA = pentaethylenehexamine; PXDA = polyxylylenediamine | | | | | | |

**Table 2. Activity of cross-linked enzyme aggregates after washing and resuspension with pentaethylenehexamine as diamine**

| | **mM PEHA** | **% CLEA* activity yield** | **U/mg** |
|---|---|---|---|
| **1** | 2.5 | 32 | 2,79 |
| **2** | 5 | 31 | 4,25 |
| **3** | 7.5 | 32 | 3,76 |
| **4** | 10 | 38 | 3,95 |
| **5** | 12.5 | 37 | 3,16 |
| **6** | 15 | 37 | 2,50 |

| | | | |
|---|---|---|---|
| *CLEA= cross-linked enzyme aggregate | | | |

### Example 4: preparation I of cross-linked laccase enzyme aggregate (glutaraldehyde and pentaethylenediamine)

1 g laccase (Trametes versicolor, Wacker Chemie) was. dissolved in 40 ml 100 mM sodium acetate buffer pH 4.5 at 4°C. After centrifugation 36 g polyethylene glycol (8000k) was added to the supernatant at room temperature. After stirring for 30 minutes, 2.873 ml chilled (4°C) glutaraldehyde (25% solution) was added dropwise. The obtained solution was stirred for 5 minutes at room temperature. Subsequently 7.4 ml pentaethylenediamine (PEHA) (as 100 mM solution, pH 4.5) was added all at once. The mixture was stirred slowly for 3 hours at room temperature. Subsequently, 200 ml water was added and the cross-linked enzyme aggregate was centrifuged. The pellet was resuspended in 9 ml 100 mM sodium acetate buffer pH 4.5 containing 10% polyethylene glycol (8000k), and frozen.

### Example 5: Working up the cross-linked enzyme aggregates by means of a drying step

The cross-linked enzyme aggregate was prepared as in Example 4. On completion of the cross-linking, the cross-linked enzyme aggregate suspension was washed 3x (centrifuged and decanted) with a same volume of demineralised water to wash out all the soluble components. The pellet was then resuspended in acetone, another water soluble solvent would also be suitable, centrifuged and resuspended in diethylether (a comparable solvent would also be suitable). After.centrifugation, the cross-linked enzyme aggregate was free of salts or other components. It was storable in the ether as suspension or the ether could be evaporated to isolate the cross-linked enzyme aggregate in the form of dry powder.

### Example 6: preparation II of cross-linked laccase enzyme aggregate (periodate oxidation and pentaethylenediamine)

1 g of laccase (Trametes versicolor, Wacker Chemie). was dissolved in 10 ml 100 mM sodium acetate buffer pH 4.5 at 4°C. After centrifugation, 10 ml 100 mM sodium metaperiodate was added to the supernatant. After incubating for 1 hour at 4°C, 18 g polyethylene glycol (8000k) was added to the supernatant at room temperature. After stirring for 30 minutes, 3.7 ml pentaethylenediamine (PEHA) (as 100 mM solution, pH 4.5) was added all at once. The obtained solution was stirred for 2 hours at room temperature. Then, 4 ml chilled (4°C) sodium cyanoborohydride (100 mM solution) was added.

The mixture was slowly stirred for 1 hour at room temperature. Then 200 ml water was added and the cross-linked enzyme aggregate was centrifuged. The pellet was resuspended in 10 ml 100 mM sodium acetate buffer pH 4.5 containing 10% polyethylene glycol (8000k), and frozen.

### Example 7: preparation of cross-linked laccase enzyme aggregate III (periodate oxidation and ammonia)

1 g laccase (Trametes versicolor, Wacker Chemie) was dissolved in 10 ml 100 mM sodium acetate buffer pH 4.5, at 4°C. After centrifugation, 10 ml 100 mM sodium metaperiodate was added to the supernatant. After incubation for 1 hour at 4°C, 10 g ammonium sulphate was added to the supernatant at room temperature and the pH was adjusted to 8.5. The mixture was then stirred for 2 hours at room temperature at pH 8.5. Then 4 ml chilled (4°C) sodium cyanoborohydride (100 mM solution) was added. The mixture was stirred slowly for 1 hour at room temperature. Then 200 ml water was added and the cross-linked enzyme aggregate was centrifuged. The pellet was resuspended in 10 ml 100 mM sodium acetate buffer pH 4.5 containing 10% polyethylene glycol (8000k) and, frozen.

### Example 8: the stability of laccase in the presence of the nitrosonium salt of TEMPO

A solution of 0.5 mg/ml laccase in 0.2 M succinate buffer pH 6 was prepared and the nitrosonium salt of TEMPO was added to give a final concentration of 32 mM. The solution was incubated and the residual enzyme activity was determined. From the relationship between the activity and time, the half-life of laccase in the presence of the nitrosonium salt of TEMPO was calculated. The same experiment was conducted with the cross-linked enzyme aggregate of Example 4 (preparation I of cross-linked laccase enzyme aggregate) in the same buffer and in the presence of 32 mM of the nitrosonium salt of TEMPO.

**Table 3. Half-life of cross-linked laccase enzyme aggregates**

| Enzyme | Half-life in the presence of the nitrosonium salt of TEMPO |
|---|---|
| Free Laccase | 15 minutes |
| Laccase-CLEA* Example 4 | 116 minutes |

| | |
|---|---|
| *CLEA= cross-linked enzyme aggregates | |

### Example 9: The stability of cross-linked laccase enzyme aggregate at 55°C

A solution of 0.5 mg/ml laccase (T. versicolor) in 40 mM sodium acetate buffer pH 4.5 and a solution of 0.5 mg/ml cross-linked enzyme aggregate from Example 4 in the same buffer was incubated at 55°C and the activity was determined at regular intervals. From the activity decrease in time it was possible to determine the half-life under these conditions. The half-life of laccase was shown to be approximately 11 hours, and that of the cross-linked enzyme aggregate from Example 4 approximately 40 hours.

### Example 10: Oxidation of starch

Starch solutions were prepared by gelling Lintner potato starch (Sigma S-2630) in water. For each experiment a solution of 16 g gelled starch in 800 ml 0.1 M succinate buffer pH 5.6 was prepared. To this solution 3.2 g TEMPO was added. (Under certain circumstances, TEMPO forms a precipitate with starch, which dissolves during the process). In each experiment, 30% of the total amount of enzyme units was added at the beginning of the reaction. During the first 6 hours of the reaction, the remaining 70% of the enzyme units was added in 6 aliquots per hour. The reaction temperature was 37°C. The pH was kept constant using a pH stat. The conversion of starch was measured by means of online analysis of the hydroxide consumption. The degree of conversion is defined as the percentage of C6-hydroxyl groups converted to carboxylic acid groups.

**Table 4. Stability of cross-linked enzyme aggregates from Example 4.**

| Enzyme | Total of units used | Degree of conversion after 24 hours |
|---|---|---|
| Laccase | 2000 | 61% |
| Laccase | 1700 | 51% |
| Cross-linked enzyme aggregate from Example 4 | 1377 | 64% |

The results show that considerably fewer units are needed when the cross-linked enzyme aggregate from Example 4 is used instead of soluble laccase.

**Table 5. Diamine selection for periodate-oxidised Candida antarctica lipase B.**

| **Pellet structure** | **Diamine (10 mM)** | **% Yield CLEA*** |
|---|---|---|
| thin | EDA | 53 |
| thin | P1,2DA | 46 |
| thin | PDA | 64 |
| thin | HMDA | 85 |
| thin | XDA | 91 |
| flocculent | PXDA | 94 |
| very flocculent | PEHA | 91 |
| thin | Lysine | 71 |
| thin | Lysine ethylester | 62 |

| | | |
|---|---|---|
| *CLEA= cross-linked enzyme aggregate EDA = 1,2 diamino ethane; P1,2DA = 1,2-propanediamine PDA = 1,3-propanediamine; HMDA = 1,6-hexamethylenediamine; XDA = xylenediamine; PXDA = polyxylylenediamine; PEHA = pentaethylenehexamine; | | |

**Table 6. Diamine optimisation for periodate-oxidised Candida antarctica lipase B.**

| **Diamine** | **Concentration mM** | **% Yield CLEA*** |
|---|---|---|
| HMDA | 5 | 76 |
| HMDA | 10 | 82 |
| HMDA | 15 | 83 |
| PXDA | 5 | 82 |
| PXDA | 10 | 85 |
| PXDA | 15 | 79 |
| PEHA | 5 | 80 |
| PEHA | 10 | 62 |
| PEHA | 15 | 67 |

| | | |
|---|---|---|
| *CLEA= cross-linked enzyme aggregate | | |

### Example 11: Lipase cross-linked enzyme aggregate preparation II (periodate oxidation and polyxylylenediamine)

An amount of 150 ml 100 mM sodium metaperiodate was added to 150 ml lipase (CaLB, Novozyme 525F) and the mixture was kept for 1 hour at room temperature. Subsequently 1.3 grams of potassium hydrogen phosphate was added and the pH was adjusted to 7.8 with diluted phosphoric acid. Then 270 g polyethylene glycol (8000k) was added and the mixture was stirred for 5 minutes at room temperature, after which 57 ml polyxylylenediamine (100 mM, pH 7,8) solution was added all at once. After 10 minutes, 57 ml chilled (4.°C) sodium cyanoborohydride (100 mM solution) was added.
The mixture was slowly stirred for 1 hour at room temperature. After that, 285 ml water was added and stirred for 1 more hour. Then the cross-linked enzyme aggregate was centrifuged. The pellet was washed thrice with 400 ml demineralised water (centrifuging, decanting), once with 300 ml acetone, once with 150 ml acetone and once with 150 ml diethylether. After evaporating the ether, the pellet was obtained in the form of dry powder.

**Table 7. Activity of Candida antarctica lipase B formulations in organic media.**

| | Hydrolytic activity^{a} | Deesterification^{b} |
|---|---|---|
| Free lipase | 22000 | - |
| Novozyme | 7300 | 250 |
| CLEA-example K not reduced | 3000 | 11 |
| CLEA-example K reduced | 38000 | 50 |
| CLEA-example L | 31000 | 1500 |

| | | |
|---|---|---|
| a) Tributyrin units/gram: 5 vol.% tributyrine in 40 mM Tris buffer, pH 7.5; 40°C b) Phenylethylamine 41 mM; n-butyl methoxyacetaat 34 mM; 12 mg/ml, Mol. sieve 4A, 40°C | | |

### Example 12: Lipolase cross-linked enzyme aggregate preparation (periodate oxidation and polyxylylenediamine)

An amount of 100 ml 300 mM sodium metaperiodate was added to 100 ml lipolase (Thermomyces Lanuginosa, Novozyme Lipolase® 100L) and the mixture was kept for 1 hour at room temperature. Subsequently, 0.6 grams of potassium hydrogen phosphate was added and the pH was adjusted to 6.6 with diluted phosphoric acid. Then 160 g polyethylene glycol (8000k) was added and the mixture was stirred for 15 minutes at room temperature, after which 35 ml polyxylylenediamine(100 mM) solution was added all at once. After 10 minutes, 35 ml chilled (4°C) sodium cyanobo-rohydride (100 mM solution) was added. The mixture was slowly stirred for 1 hour at room temperature. After that, 250 ml water was added and stirred for 1 more hour. Then the cross-linked enzyme aggregate was centrifuged. The pellet was washed thrice with 400 ml demin-eralised water (centrifuging, decanting), once with 300 ml acetone, once with 150 ml acetone and once with 150 ml die-thylether. After evaporating the ether, the pellet was ob-tained in the form of dry powder. The yield was 3.95 g with an activity of 189000 tributyrin units per gram.

### Example 13: Lipolase cross-linked enzyme aggregate preparation (periodate oxidation and polyxylylenediamine with a silica coating)

An amount of 100 ml 300 mM sodium metaperiodate was added to 100 ml lipolase (Thermomyces Lanuginosa, Novozyme Lipolase® 100L) and the mixture was kept for 1 hour at room temperature. Subsequently, 0.6 grams of potassium hydrogen phosphate was added and the pH was adjusted to 6.6 with diluted phosphoric acid. Then 160 g polyethylene glycol (8000k) was added and the mixture was stirred for 15 minutes at room temperature, after which 35 ml polyxylylenediamine (100 mM) solution was added all at once. After 10 minutes, 35 ml chilled (4°C) sodium cyanoborohydride (100 mM solution) was added. The mixture was slowly stirred for 1 hour at room temperature. Then sodium fluoride was added (25 ml 1 M solution) and subsequent 10 ml of (EtO)₄Si. After stirring overnight, 250 ml water was added and the suspension was filtered over a P4 glass filter. The pellet was washed thrice with 400 ml demineralised water, once with 300 ml acetone, once with 150 ml acetone and once with 150 ml diethylether. After evaporating the ether, the pellet was obtained in the form of dry powder. The yield was 6 g with an activity of 650000 tributyrin units per gram.

### Example 14: Cross-linking of enzyme aggregates with the addition of a carrier

In order to obtain a good bond between the aggregate and the carrier, silica (Davisil 644, 100-200 mesh, 150A) was pre-treated with 3-aminopropyltriethoxysilane (as described in WO 03/031610). This causes amino groups to be formed on the carrier which, as described, are able to react with the aldehyde groups on the enzyme. To 1 g of this pre-treated silica, 5 ml demineralised water and 12 ml of the periodate-treated and with polyethylene glycol aggregated enzyme (CaLB, Novozyme 525F) from Example 12 was added. It is also possible to add the silica before aggregation. After stirring for 15 minutes, 1.7 ml PXDA (100 mM) was added and the mixture stirred for 2 hours. Subsequently, it was reduced for 1 hour using 1.7 ml 100 mM NaCNBH₃. The thus obtained cross-linked enzyme aggregate was washed on a glass filter with water, acetone and ether. The activity of the solid (900 mg) in the tributyrine assay was 2400 units per gram. This corresponds with approximately 10% enzyme per gram of carrier material.

### Example 15: Cross-linking of enzyme aggregates with the addition of a carrier II

To 2.5 g of Sepabeads® FP serie, EC-EA300 (Resin-dion, Italy), 2 ml demineralised water and 2 ml of the periodate-treated CaLB from Example 12 was added. The pH was adjusted to 7.5. After shaking for 1 hour 15 ml 2-propanol was added and subsequently, it was reduced for 1 hour using 2 ml 100 mM NaCNBH3. The thus obtained particles were washed on a glass filter with water, acetone and ether. The activity of the solid (900 mg) in the tributyrin assay was 3000 units per gram.

## Claims

1. A method for the preparation of cross-linked enzyme aggregates, **characterised by** the following steps:
i. generating aldehyde groups on enzyme molecules that may be dissolved in a solvent, by the addition of an oxidant: selected from the group of periodates of alkaline-earth metals;
ii. precipitating the enzyme molecules using a suitable precipitation agent;
iii. cross-linking the precipitated enzyme molecules provided with aldehyde groups, using an amine composition, yielding cross-linked enzyme aggregates with improved properties.

2. A method according to claim 1, **characterised in that** a reducing agent is added in step iii.

3. A method according to claim 2, **characterised in that** the reducing agent is selected from the group of NaCNBH₃ or NaBH₄.

4. A method according to claims 1-3, **characterised in that** an alkoxysilane is added in step iii.

5. Method according to claim 4, **characterised in that** the alkoxysilane is selected from the group of (MeO)₄Si, (EtO)₄Si, Me(MeO)₃Si and propyl(MeO)₃Si.

6. A method according to one or more of the claims 1-5, **characterised in that** in step iii. ammonia is used as amine compound.

7. A method according to one or more of the claims 1-5, **characterised in that** in step iii. a di- or polyamine is used as amine compound.

8. A method according to one or more of the claims 1-5, **characterised in that** the amine compound in step iii. is derived from the precipitation agent.

9. A method according to claim 8, **characterised in that** the precipitation agent is an ammonium compound and the amine compound is ammonia, the method being performed at a pH of 8-9.5.

10. A method according to claim 9, **characterised in that** the ammonium compound is ammonium sulphate.

11. A method according to one of the preceding claims, **characterised in that** a carrier. material is added in step i.

12. A method according to claim 11, **characterised in that** the carrier material is silica.

13. A method according to one of the preceding claims, **characterised in that** after step iii., the cross-linked enzyme aggregates are washed with demineralised water, followed by a drying step.

14. A method according to claim 13, **characterised in that** the drying step is carried out by means of treatment with an organic solvent.

15. A method according to claim 14, **characterised in that** the organic solvent is selected from the group of water-miscible solvents.

16. A method according to claim 14 or 15, **characterised in that** the organic solvent is subsequently removed by the addition of ether, followed by evaporation.

17. A method according to one of the preceding claims, **characterised in that** the enzyme molecules are selected from the group of laccase, lipase, protease, esterase, oxynitrilase, nitrilase, aminoacylase, penicillin acylase, lyase, oxidase or reductase molecules.

## Patentansprüche

1. Verfahren für die Herstellung vernetzter Enzymaggregate, **gekennzeichnet durch** die folgenden Schritte:
i. Erzeugen von Aldehydgruppen an Enzymmolekülen, die in einem Lösungsmittel gelöst sein können, **durch** die Zugabe eines Oxidationsmittels, das aus der Gruppe der Periodate von Alkali-Erdmetallen ausgewählt ist;
ii. Fällen der Enzymmoleküle unter Verwendung eines geeigneten Fällungsmittels;
iii. Vernetzen der gefällten Enzymmoleküle, die mit Aldehydgruppen versehen sind, unter Verwendung einer Aminzusammensetzung, wodurch vernetzte Enzymaggregate mit verbesserten Eigenschaften erhalten werden.

2. Verfahren nach Anspruch 1, **dadurch gekennzeichnet, dass** in Schritt iii. ein Reduktionsmittel hinzugefügt wird.

3. Verfahren nach Anspruch 2, **dadurch gekennzeichnet, dass** das Reduktionsmittel aus der Gruppe NaCNBH₃ oder NaBH₄ ausgewählt ist.

4. Verfahren nach den Ansprüchen 1-3, **dadurch gekennzeichnet, dass** in Schritt iii. ein Alkoxysilan hinzugefügt wird.

5. Verfahren nach Anspruch 4, **dadurch gekennzeichnet, dass** das Alkoxysilan aus der Gruppe (MeO)₄Si, (EtO)₄Si, Me(MeO)₃Si und Propyl(MeO)₃Si ausgewählt ist.

6. Verfahren nach einem oder mehreren der Ansprüche 1-5, **dadurch gekennzeichnet, dass** in Schritt iii. Ammoniak als Aminverbindung verwendet wird.

7. Verfahren nach einem oder mehreren der Ansprüche 1-5, **dadurch gekennzeichnet, dass** in Schritt iii. ein Di- oder Polyamin als Aminverbindung verwendet wird.

8. Verfahren nach einem oder mehreren der Ansprüche 1-5, **dadurch gekennzeichnet, dass** die Aminverbindung in Schritt iii. von dem Fällungsmittel abgeleitet ist.

9. Verfahren nach Anspruch 8, **dadurch gekennzeichnet, dass** es sich bei dem Fältungsmittel um eine Ammoniumverbindung handelt und es sich bei der Aminverbindung um Ammoniak handelt, wobei das Verfahren bei einem pH-Wert von 8-9,5 durchgeführt wird.

10. Verfahren nach Anspruch 9, **dadurch gekennzeichnet, dass** es sich bei der Ammoniumverbindung um Ammoniumsulfat handelt.

11. Verfahren nach einem der vorhergehenden Ansprüche, **dadurch gekennzeichnet, dass** in Schritt i. ein Trägermaterial hinzugefügt wird.

12. Verfahren nach Anspruch 11, **dadurch gekennzeichnet, dass** es sich bei dem Trägermaterial um Silica handelt.

13. Verfahren nach einem der vorhergehenden Ansprüche, **dadurch gekennzeichnet, dass** nach Schritt iii. die vernetzten Enzymaggregate mit demineralisiertem Wasser gewaschen werden, gefolgt von einem Trocknungsschritt.

14. Verfahren nach Anspruch 13, **dadurch gekennzeichnet, dass** der Trocknungsschritt durch Behandlung mit einem organischen Lösungsmittel durchgeführt wird.

15. Verfahren nach Anspruch 14, **dadurch gekennzeichnet, dass** das organische Lösungsmittel aus der Gruppe wassermischbarer Lösungsmittel ausgewählt ist.

16. Verfahren nach Anspruch 14 oder 15, **dadurch gekennzeichnet, dass** das organische Lösungsmitten anschließend durch die Zugabe von Ether, gefolgt von Verdunstung, entfernt wird.

17. Verfahren nach einem der vorhergehenden Ansprüche, **dadurch gekennzeichnet, dass** die Enzymmoleküle aus der Gruppe von Laccase-, Lipase-, Protease-, Esterase-, Oxynitrilase-, Nitrilase-, Aminoacylase-, Penicillinacylase-, Lyase-, Oxidase- oder Reduktasemolekülen ausgewählt sind.

## Revendications

1. Méthode de préparation d'agrégats d'enzyme réticulée, **caractérisée par** les étapes suivantes :
i. génération de groupements aldéhyde sur des molécules d'enzyme qui peuvent être solubilisées dans un solvant, par l'addition d'un oxydant choisi dans le groupe constitué par les périodates de métaux alcalino-terreux ;
ii. précipitation des molécules d'enzyme en utilisant un agent de précipitation adapté ;
iii. réticulation des molécules d'enzyme précipitées pourvues de groupements aldéhyde, en utilisant une composition d'amine, conduisant à des agrégats d'enzyme réticulée ayant des propriétés améliorées.

2. Méthode selon la revendication 1, **caractérisée en ce qu'**un agent réducteur est ajouté dans l'étape iii.

3. Méthode selon la revendication 2, **caractérisée en ce que** l'agent réducteur est choisi dans le groupe constitué par NaCNBH₃ ou NaBH₄.

4. Méthode selon les revendications 1-3, **caractérisée en ce qu'**un alcoxysilane est ajouté dans l'étape iii.

5. Méthode selon la revendication 4, **caractérisée en ce que** l'alcoxysilane est choisi dans le groupe constitué par (MeO)₄Si, (EtO)₄Si, Me(MeO)₃Si et propyl(MeO)₃Si.

6. Méthode selon une ou plusieurs des revendications 1-5, **caractérisée en ce que** dans l'étape iii., de l'ammoniac est utilisé comme composé aminé.

7. Méthode selon une ou plusieurs des revendications 1-5, **caractérisée en ce que** dans l'étape iii., une di- ou polyamine est utilisée comme composé aminé.

8. Méthode selon une ou plusieurs des revendications 1-5, **caractérisée en ce que** le composé aminé dans l'étape iii. est dérivé de l'agent de précipitation.

9. Méthode selon la revendication 8, **caractérisée en ce que** l'agent de précipitation est un composé d'ammonium, et le composé aminé est l'ammoniac, la méthode étant mise en oeuvre à un pH de 8-9,5.

10. Méthode selon la revendication 9, **caractérisée en ce que** le composé d'ammonium est le sulfate d'ammonium.

11. Méthode selon l'une des revendications précédentes, **caractérisée en ce qu'**un matériau support est ajouté dans l'étape i.

12. Méthode selon la revendication 11, **caractérisée en ce que** le matériau support est de la silice.

13. Méthode selon l'une des revendications précédentes, **caractérisée en ce qu'**après l'étape iii., les agrégats d'enzyme réticulée sont lavés par de l'eau déminéralisée, suivi d'une étape de séchage.

14. Méthode selon la revendication 13, **caractérisée en ce que** l'étape de séchage est effectuée au moyen d'un traitement par un solvant organique.

15. Méthode selon la revendication 14, **caractérisée en ce que** le solvant organique est choisi dans le groupe constitué par les solvants miscibles avec l'eau.

16. Méthode selon la revendication 14 ou 15, **caractérisée en ce que** le solvant organique est ensuite éliminé par l'addition d'éther, suivi d'une évaporation.

17. Méthode selon l'une des revendications précédentes, **caractérisée en ce que** les molécules d'enzyme sont choisies dans le groupe constitué par les molécules de laccase, de lipase, de protéase, d'estérase, d'oxynitrilase, de nitrilase, d'aminoacylase, de pénicilline acylase, de lyase, d'oxydase ou de réductase.
